# EUROPÄISCHE PATENTSCHRIFT

(11) **EP 1 803 435 B1**
(45) Veröffentlichungstag und Bekanntmachung des Hinweises auf die Patenterteilung: **08.06.2016**
(21) Anmeldenummer: 06025396.0
(22) Anmeldetag: 08.12.2006
(51) Int. Cl.: A61Q 17/04, A61K 8/02, A61K 8/06, A61K 8/19, A61K 8/29, A61K 8/34, A61K 8/35, A61K 8/44, A61K 8/49, A61K 8/64, A61K 8/66, A61K 8/89, A61K 8/895

(54) **Kosmetische Lichtschutzzusammensetzungen auf der Basis lamellarer Emulsionen**
Cosmetic sun protection compositions based on lamelliform emulsions
Compositions cosmétiques dotées d'une protection contre les rayonnements solaires sur la base d'émulsions lamellaires

(30) Priorität: 30.12.2005 DE 102005063178
(43) Veröffentlichungstag der Anmeldung: 04.07.2007
(73) Patentinhaber: Henkel AG & Co. KGaA, 40589 Düsseldorf (DE)
(72) Erfinder: Döring, Thomas, 41542 Dormagen (DE); Anderheggen, Bernd, 42781 Haan (DE); Wadle, Armin, 40699 Erkrath (DE)

(56) Entgegenhaltungen:
- EP-A2- 1 077 059
- EP-A2- 1 092 414
- EP-A2- 1 291 010
- WO-A1-97/21421
- WO-A1-98/44896
- WO-A1-2004/069216
- WO-A1-2006/100018
- WO-A2-01/49256
- WO-A2-2006/103037
- US-A1- 2004 091 434
- US-A1- 2004 151 673

## Beschreibung

Die Haut ist das größte Organ des Menschen. Unter ihren vielen Funktionen (beispielsweise zur Wärmeregulation und als Sinnesorgan) ist die Barrierefunktion, diejenige die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.
Die Epidermis ist ein stratifiziertes Gewebe, in dem die äußere Schicht, die Hornschicht (stratum corneum) den für die Barrierefunktion bedeutenden Teil darstellt.
Sie wird im Kontakt mit der Umwelt abgenutzt und befindet deshalb sich in einem ständigen Erneuerungsprozess, wobei nach außen kontinuierlich feine Schuppen abgegeben und von innen verhorntes Zell- und Lipidmaterial nachproduziert wird.
Das heute in der Fachwelt anerkannte Hautmodell von Elias (P. M. Elias, Structure and Function of the Stratum Corneum Permeability Barrier, Drug Dev. Res. 13, 1988, 97 - 105) beschreibt die Hornschicht als Zwei-Komponenten-System, ähnlich einer Ziegelsteinmauer (Ziegelstein-Mörtel-Modell). In diesem Modell entsprechen die Hornzellen (Corneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden. Die besondere Struktur der Hornschicht schützt einerseits die Haut und stabilisiert andererseits ihre eigene Flexibilität durch Bindung einer definierten Wassermenge.
Die Regulation des Wasser- und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei.

Die Lipide der Hornschicht bestehen im wesentlichen aus Ceramiden, freien Fettsäuren, Cholesterin sowie Cholesterinsulfat und sind über die gesamte Hornschicht verteilt. Die Zusammensetzung dieser Lipide ist für die intakte Funktion der epidermalen Barriere und damit für die Wasserundurchlässigkeit der Haut von entscheidender Bedeutung.
Bereits bei einer Reinigung der Haut mit Hilfe eines einfachen Wasserbads - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.
Bei gesunder Haut sind diese Vorgänge im allgemeinen belanglos, da die Schutzmechanismen der Haut solche leichten Störungen der oberen Hautschichten ohne weiteres kompensieren können. Aber bereits im Fall nicht-pathologischer Abweichungen vom Normalstatus, z. B. durch umweltbedingte Abnutzungsschäden bzw. Irritationen, Lichtschäden, Altershaut usw., ist der Schutzmechanismus an der Hautoberfläche gestört. Bei alter Haut beispielsweise erfolgt die regenerative Erneuerung verlangsamt, wobei insbesondere das Wasserbindungsvermögen der Hornschicht nachlässt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen.
Bei pathologisch trockener und empfindlicher Haut liegt ein Barriereschaden a priori vor. Epidermale Interzellularlipide werden fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Im Stand der Technik sind diverse Hautpflegeprodukte bekannt, die in der Lage sind, die epidermale Barrierefunktion zu verbessern. Diese Hautpflegeprodukte sind jedoch in der Regel okklusiver Natur, z. B. Vaseline und Vaseline-haltige Cremes, und weisen häufig ein klebriges Hautgefühl und/oder einen unangenehmen Tragekomfort auf. Die Salbe oder Creme stellt sozusagen eine (zweite) künstliche Barriere dar, die den Wasserverlust der Haut verhindern soll. Entsprechend leicht kann diese physikalische Barriere - beispielsweise mit Reinigungsmitteln - wieder entfernt werden, wodurch der ursprüngliche, beeinträchtigte Zustand wieder erreicht wird. Darüber hinaus kann die Hautpflegewirkung bei regelmäßiger Behandlung nachlassen. Nach dem Absetzen der Produktanwendung kehrt die Haut sehr schnell wieder in den Zustand vor Behandlungsbeginn zurück. Eine nachhaltige Produktwirkung wird in der Regel also nicht oder nur in einem eingeschränkten Maße erreicht. Um die Haut bei ihrer natürlichen Regeneration zu unterstützen und ihre physiologische Funktion zu stärken, werden den topischen Präparaten in neuerer Zeit zunehmend Interzellularlipidmischungen, wie Ceramide oder Ceramidanaloga, zugesetzt, die von der Haut zum Wiederaufbau der natürlichen Barriere verwendet werden sollen. Allerdings handelt es sich bei diesen Lipiden zumeist um sehr teure Rohstoffe. Zudem ist ihre Wirkung meist geringer als erhofft.
Dem Stand der Technik mangelt es demnach an Zubereitungen, die die Barrierefunktion und die Hydratation der Hornschicht positiv beeinflussen und die physikalisch-chemischen Eigenschaften der Hornschicht und insbesondere der Lamellen aus Interzellularlipiden stärken bzw. sogar wiederherstellen.
Jüngere Untersuchungen haben gezeigt, dass unter trockenen Bedingungen oder unter UV-Bestrahlung die Barrierelipidsynthese und -sekretion in der äußeren Granularkeratinozytenschicht stimuliert wird (J. Invest. Dermatol. 111, 858 - 863 (1998), J.Invest. Dermatol. 109, 783-787 (1997), J. Lipid Res. 32, 1151 - 1158 (1991)).
Aus dem Stand der Technik ist bekannt, dass lamellare Emulsionen die gestörte Lamellarität der Hautbarrierelipidstruktur wiederherstellen können (siehe WO 98/44896 A1) und den Feuchtigkeitshaushalt der Haut verbessern. In zahlreichen Untersuchungen wurde beobachtet, dass bestimmte UV-Filter die Haut austrocknen können beziehungsweise den hautbefeuchtenden Effekt von kosmetischen Emulsionen mindern können. Weiterhin wurde beobachtet, dass lamellare Emulsionen, insbesondere solche, die auf linearen C₂₀-C₄₀-Fettalkoholen basieren, in Gegenwart von bestimmten UV-Filtern ihre lamellaren Strukturen teilweise oder ganz verlieren können. Weiterhin neigen lamellare Emulsionen in Gegenwart von bestimmten UV-Filtern zur beschleunigten Phasentrennung. Dieses Problem besteht insbesondere, wenn gleichzeitig spezifische Wirkstoffe eingesetzt werden, insbesondere Peptide und Pflanzenextrakte.

Basierend auf diesen Beobachtungen, war es eine Aufgabe der vorliegenden Erfindung, topische Zusammensetzungen zu entwickeln, die die Barrierefunktion der Haut, beispielsweise gegenüber dem transepidermalen Wasserverlust, stärken und gleichzeitig einen wirksamen UV-Schutz bieten. Eine weitere Aufgabe der vorliegenden Erfindung war es, topische Zusammensetzungen zu entwickeln, die die Barrierefunktion der Haut stärken, einen wirksamen UV-Schutz bieten und gleichzeitig lagerstabil sind. Eine weitere Aufgabe der vorliegenden Erfindung war es, topische Zusammensetzungen zu entwickeln, die die Barrierefunktion der Haut stärken, einen wirksamen UV-Schutz bieten und gleichzeitig ein gutes Hautgefühl aufweisen und sich insbesondere nicht klebrig anfühlen.

Überraschenderweise wurden nun bestimmte UV-Filter-Kombinationen gefunden, die die lamellaren flüssig-kristallinen Strukturen in einer lamellaren Öl-in-Wasser-Emulsion nicht stören. Bei den erfindungsgemäßen lamellaren Lichtschutzemulsionen ist weiterhin besonders vorteilhaft, dass sie sowohl mindestens einen lipidlöslichen UV-Filter in der Öl-phase als auch mindestens einen wasserlöslichen UV-Filter in der Wasserphase enthalten. Ein weiterer Vorteil der erfindungsgemäßen lamellaren Lichtschutzemulsionen ist, dass sie sehr gute feuchtigkeitsspendende Eigenschaften aufweisen. Untersuchungen an nicht erfindungsgemäßen Lichtschutzemulsionen ohne lamellare flüssig-kristalline Strukturen haben nämlich immer wieder gezeigt, dass ein Gehalt an UV-Filtern, seien es nun organische oder anorganische UV-Filter oder Lichtschutz-Pigmente, die feuchtigkeitsspendenden Eigenschaften kosmetischer Zusammensetzungen, insbesondere kosmetischer Emulsionen, beeinträchtigen kann. Dies ist insbesondere für die mittlerweile beim Verbraucher immer beliebter werdenden Tagescremes mit Lichtschutzfaktor nachteilig, da derartige Cremes zwar zum Schutz der Haut vor vorzeitiger Lichtalterung beitragen, gleichzeitig aber der Haut weniger Feuchtigkeit spenden als Cremes ohne Lichtschutzfaktor. Die erfindungsgemäßen Zusammensetzungen führen aus diesem Dilemma heraus und ermöglichen eine hochwertige Tagespflege, die einen wirksamen Schutz der Haut vor vorzeitiger Lichtalterung mit einer guten hautbefeuchtenden Produktleistung verbinden. Sie lösen die erfindungsgemäß gestellten Aufgaben und stärken die Barrierefunktion der Haut, insbesondere gegenüber dem transepidermalen Wasserverlust. Gleichzeitig weisen sie ein angenehmes, nicht-okklusives und nicht-klebriges Hautgefühl auf. Sie bewirken eine lang anhaltende Stärkung der Barrierefunktion der Haut.

Gegenstand der vorliegenden Erfindung ist daher eine Öl-in-Wasser-Emulsion, die mindestens ein Alkyl- und/oder Alkoxy-substituiertes Dibenzoylmethanderivat, mindestens einen wasserlöslichen UV-Filter, ausgewählt aus Phenylbenzimidazolsulfonsäure und Phenyldibenzimidazoltetrasulfonsäure und deren physiologisch verträglichen Salzen und/oder Estern, mindestens einen weiteren UV-Filter, ausgewählt aus Polysilicone-15, s-Triazinderivaten und mindestens einem anorganischen UV-Schutz-Pigment, und mindestens einen primären, linearen, gesättigten Fettalkohol mit einer Kettenlänge von 20 - 40 Kohlenstoffatomen enthält, wobei die Emulsion lamellare flüssig-kristalline Strukturen aufweist und frei ist von Salicylsäureestern.

Erfindungsgemäße lamellare Öl-in-Wasser-Emulsionen sind dadurch gekennzeichnet, dass mindestens ein primärer, linearer, gesättigter Fettalkohol mit einer Kettenlänge von 20 - 40 Kohlenstoffatomen enthalten ist. In Gegenwart dieses mindestens einen Fettalkohols und den erfindungsgemäßen UV-Filterkombinationen bei gleichzeitiger Abwesenheit von Salicylsäureestern bilden sich lamellare flüssig-kristalline Phasen aus. Beispiele für erfindungsgemäß ausgeschlossene Salicylsäureester sind die Verbindungen mit der INCI-Bezeichnung Octyl Salicylate, Ethylhexyl Salicylate und Homosalate.
Besonders bevorzugte erfindungsgemäße lamellare Öl-in-Wasser-Emulsionen sind dadurch gekennzeichnet, dass sie Behenylalkohol und/oder Arachidylalkohol, insbesondere als technische Mischung beider Alkohole, enthalten. Die auf diesen längerkettigen Fettalkoholen basierenden lamellaren Phasen sind weniger störanfällig gegenüber anderen Bestandteilen der Emulsion. Außerordentlich bevorzugt sind daher lamellare Öl-in-Wasser-Emulsionen, die mindestens ein Alkyl- und/oder Alkoxy-substituiertes Dibenzoylmethanderivat, mindestens einen wasserlöslichen UV-Filter, ausgewählt aus Phenylbenzimidazolsulfonsäure und Phenyldibenzimidazoltetrasulfonsäure und deren physiologisch verträglichen Salzen und/oder Estern, mindestens einen weiteren UV-Filter, ausgewählt aus Polysilicone-15, s-Triazinderivaten und mindestens einem anorganischen UV-Schutz-Pigment, enthalten, wobei die Emulsionen frei sind von Salicylsäureestern.
Besonders bevorzugte erfindungsgemäße Zusammensetzungen enthalten mindestens einen primären, linearen, gesättigten Fettalkohol mit einer Kettenlänge von 20 - 40 Kohlenstoffatomen in einer Gesamtmenge von 0,5 - 15 Gew.-%, bevorzugt 0,8 - 10 Gew.-%, besonders bevorzugt 1-8 Gew.-% und außerordentlich bevorzugt 1,5 - 5 Gew.-%, jeweils bezogen auf das Gewicht der gesamten Zusammensetzung.
Bevorzugte lineare primäre Fett- oder Wachsalkohole mit einer Kettenlänge der Alkylgruppe von 20 - 40 Kohlenstoffatomen sind ausgewählt aus Arachidylalkohol (1-Eicosanol), Behenylalkohol, Lignocerylalkohol (1-Tetracosanol, C₂₄H₅₀O), Cerylalkohol (C₂₆H₅₄O), Myricylalkohol (1-Triacontanol, C₃₀H₆₂O) und Melissylalkohol (C₃₁H₆₄O, 1-Hentriacontanol). Besonders bevorzugt sind Arachidylalkohol und Behenylalkohol. In einer bevorzugten Ausführungsform der Erfindung ist Arachidylalkohol in Mengen von 0,05 - 5 Gew.-%, besonders bevorzugt 0,1 - 4 Gew.-%, jeweils bezogen auf das Gewicht der gesamten Zusammensetzung, enthalten. In einer weiteren bevorzugten Ausführungsform der Erfindung ist Behenylalkohol in Mengen von 0,5 - 4 Gew.-%, besonders bevorzugt 1 - 2 Gew.-%, jeweils bezogen auf das Gewicht der gesamten Zusammensetzung, enthalten. In einer weiteren besonders bevorzugten Ausführungsform der Erfindung ist eine Mischung aus Arachidylalkohol und Behenylalkohol enthalten, wobei die für die Einzelsubstanzen genannten bevorzugten Mengenangaben auch für die Mischung als bevorzugt gelten.
Die Ausbildung lamellarer Flüssigkristallphasen kann durch die Anwesenheit von partiell oder ganz neutralisierten C₆ - C₃₀-Fettsäuren behindert oder sogar unterdrückt werden. Besonders bevorzugte erfindungsgemäße lamellare Öl-in-Wasser-Emulsionen sind daher dadurch gekennzeichnet, dass sie von partiell oder ganz neutralisierten C₆-C₃₀-Fettsäuren frei sind.
Weitere besonders bevorzugte erfindungsgemäße lamellare Öl-in-Wasser-Emulsionen sind dadurch gekennzeichnet, dass sie von 4-Methylbenzylidencampher frei sind.
Die Anwesenheit lamellarer Flüssigkristallphasen schließt aus, dass es sich bei den erfindungsgemäßen lamellaren Öl-in-Wasser-Emulsionen um Mikroemulsionen oder PIT-Emulsionen handelt. Besonders bevorzugte erfindungsgemäße lamellare Öl-in-Wasser-Emulsionen sind daher dadurch gekennzeichnet, dass sie keine Mikroemulsion und/oder keine PIT-Emulsion sind.

Erfindungsgemäß bevorzugte Alkyl- und/oder Alkoxy-substituierte Dibenzoylmethanderivate sind dadurch gekennzeichnet, dass einer der Phenylreste mit mindestens einer Alkylgruppe, ausgewählt aus Methyl, Ethyl, n-Propyl, Isopropyl, n-Butyl, sec-Butyl, tert.-Butyl, n-Pentyl, Isopentyl, Neopentyl, 2-Ethylhexyl, und der andere Phenylrest mit mindestens einer Alkoxy-Gruppe, ausgewählt aus Methoxy, Ethoxy, n-Propoxy, Isopropoxy, n-Butoxy, sec-Butoxy, tert.Butoxy, n-Pentoxy, Isopentoxy, Neopentoxyl, 2-Ethylhexoxy, substituiert ist. Besonders bevorzugte Substituenten sind Isopropyl, tert.-Butyl und Methoxy. Erfindungsgemäß bevorzugte Alkyl- und/oder Alkoxy-substituierte Dibenzoylmethanderivate sind ausgewählt aus 2-Methyldibenzoylmethan, 4-Methyldibenzoylmethan, 4-Isopropyldibenzoylmethan, 4-tert-Butyldibenzoylmethan, 2,4- Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-Methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan. Besonders bevorzugt ist 4-tert-Butyldibenzoylmethan mit der INCI-Bezeichnung Butyl Methoxydibenzoylmethane, ein öllöslicher organischer Lichtschutzfilter, der z. B. unter dem Handelsnamen PARSOL^{®} 1789 von Roche oder unter Eusolex^{®} 9020 von Merck KGaA erhältlich ist. Weitere bevorzugte erfindungsgemäße lamellare Öl-in-Wasser-Emulsionen sind dadurch gekennzeichnet, dass das Alkyl- und/oder Alkoxy-substituierte Dibenzoylmethanderivat a) ausgewählt ist aus tert.-Butylmethoxydibenzoylmethan.
Weitere besonders bevorzugte erfindungsgemäße lamellare Öl-in-Wasser-Emulsionen sind dadurch gekennzeichnet, dass das Alkyl- und/oder Alkoxy-substituierte Dibenzoylmethanderivat a) in Gesamtmengen von 0,5 - 2,5 Gew.-%, bevorzugt 1,5 - 2,0 Gew.-%, jeweils bezogen auf das Gesamtgewicht der Emulsion, enthalten ist.

Erfindungsgemäß bevorzugte lamellare Öl-in-Wasser-Emulsionen sind dadurch gekennzeichnet, dass das Salz der Phenylbenzimidazolsulfonsäure und/oder Phenyldibenzimidazoltetrasulfonsäure ausgewählt ist aus den Natrium-, Kalium-, Ammonium-, Trialkylammonium- und Ethanolamin-Salzen. Die Natriumsalze sind besonders bevorzugt.
Weitere besonders bevorzugte erfindungsgemäße lamellare Öl-in-Wasser-Emulsionen sind dadurch gekennzeichnet, dass mindestens ein wasserlöslicher UV-Filter, ausgewählt aus Phenylbenzimidazolsulfonsäure und Phenyldibenzimidazoltetrasulfonsäure und deren physiologisch verträglichen Salzen und/oder Estern, in Gesamtmengen von 0,5 - 6,0 Gew.-%, bevorzugt 1,0 - 4,0 Gew.-% und außerordentlich bevorzugt 1,5 - 2,5 Gew.-%, jeweils bezogen auf das Gesamtgewicht der Emulsion, enthalten ist.

Weitere besonders bevorzugte erfindungsgemäße lamellare Öl-in-Wasser-Emulsionen sind dadurch gekennzeichnet, dass mindestens ein Alkyl- und/oder Alkoxy-substituiertes Dibenzoylmethanderivat, mindestens einen wasserlöslichen UV-Filter, ausgewählt aus Phenylbenzimidazolsulfonsäure und Phenyldibenzimidazoltetrasulfonsäure und deren physiologisch verträglichen Salzen und/oder Estern, und als weiteren UV-Filter Polysilicone-15 enthalten, wobei die Emulsion frei ist von Salicylsäureestern. Die Substanz mit der INCI-Bezeichnung Polysilicone-15 wird auch als Dimethicodiethylbenzalmalonat, Diethylbenzylidene Malonate Dimethicone oder Diethylmalonylbenzylidene Oxypropene Dimethicone bezeichnet und ist unter dem Handelsnamen Parsol^{®} SLX von Roche Vitamins erhältlich. Die chemische Struktur ist beispielsweise in EP 709080 A2 beschrieben. Besonders bevorzugt sind die Kombinationen tert.-Butylmethoxydibenzoylmethan, Phenylbenzimidazolsulfonsäure und Polysilicone-15 sowie tert.-Butylmethoxydibenzoylmethan, Dinatriumphenylbenzimidazoltetrasulfonat und Polysilicone-15.

Weitere besonders bevorzugte erfindungsgemäße lamellare Öl-in-Wasser-Emulsionen sind dadurch gekennzeichnet, dass Polysilicone-15 in einer Menge von 0,5 - 10,0 Gew.-%, bevorzugt 2,0 - 5,0 - 7,0 Gew.-% und außerordentlich bevorzugt 1,5 - 2,5 - 3,0 Gew.-%, jeweils bezogen auf das Gesamtgewicht der Emulsion, enthalten ist.

Weitere besonders bevorzugte erfindungsgemäße lamellare Öl-in-Wasser-Emulsionen sind dadurch gekennzeichnet, dass mindestens ein Alkyl- und/oder Alkoxy-substituiertes Dibenzoylmethanderivat, mindestens einen wasserlöslichen UV-Filter, ausgewählt aus Phenylbenzimidazolsulfonsäure und Phenyldibenzimidazoltetrasulfonsäure und deren physiologisch verträglichen Salzen und/oder Estern, und als weiteren UV-Filter mindestens ein s-Triazinderivat enthalten, wobei die Emulsion frei ist von Salicylsäureestern.

Erfindungsgemäß bevorzugte s-Triazinderivate sind ausgewählt aus den in EP 570838 A1 beschriebenen s-Triazinderivaten, deren chemische Struktur durch die generische Formel (I) wiedergegeben wird, wobei R einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, darstellt, X ein Sauerstoffatom oder eine NH-Gruppe darstellt, R₁ einen verzweigten oder unverzweigten C₁-C₁₈-Alkylrest, einen C₅-C₁₂- Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel (x) bedeutet, in welcher A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen, R₃ ein Wasserstoffatom oder eine Methylgruppe darstellt, n eine Zahl von 1 bis 10 darstellt, R₂ einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, darstellt, wenn X die NH-Gruppe darstellt, und einen verzweigten oder unverzweigten C₁-C₁₈- Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel bedeutet, in welcher A einen verzweigten oder unverzweigten C₁-C₁₈- Alkylrest, einen C₅-C₁₂-Cycloalkyl- oder Arylrest darstellt, gegebenenfalls substituiert mit einer oder mehreren C₁-C₄-Alkylgruppen, R₃ ein Wasserstoffatom oder eine Methylgruppe darstellt, n eine Zahl von 1 bis 10 darstellt, wenn X ein Sauerstoffatom darstellt.
Erfindungsgemäß besonders bevorzugte lamellare Öl-in-Wasser-Emulsionen sind dadurch gekennzeichnet, dass das Triazinderivat c)ii) ausgewählt ist aus symmetrisch substituierten Triazinderivaten. Ein besonders bevorzugtes symmetrisch substituiertes s-Triazin ist 4,4',4"- (1,3,5- Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5- triazin (INCI: Ethylhexyl Triazone), das unter der Warenbezeichnung UVINUL® T 150 (BASF) vertrieben wird.
Erfindungsgemäß bevorzugt kann auch ein unsymmetrisch substituiertes s-Triazin mit der INCI-Bezeichnung Dioctylbutamidotriazone sein, das unter der Handelsbezeichnung UVASORB HEB bei Sigma 3V erhältlich ist.
Erfindungsgemäß bevorzugte s-Triazinderivate sind ferner das 2,4-Bis-{[4-(3-sulfonato)-2-hydroxy-propyloxy)- 2-hydroxy]-phenyl}-6-(4-methoxyphenyl)-1,3,5-triazin-Natriumsalz, das 2, 4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3- (2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4- (1',1',1',3',5',5',5'-Heptamethylsiloxy-2"-methyl-propyloxy)-2-hydroxy]-phenyl}-6-(4-methoxyphenyl)-1,3,5-triazin.
Weitere besonders bevorzugte erfindungsgemäße lamellare Öl-in-Wasser-Emulsionen sind dadurch gekennzeichnet, dass mindestens ein s-Triazinderivat in einer Gesamtmenge von 0,5 - 10,0 Gew.-%, bevorzugt 1,0 - 5,0 - 7,0 Gew.-% und außerordentlich bevorzugt 1,5 - 2,5 - 3,0 Gew.-%, jeweils bezogen auf das Gesamtgewicht der Emulsion, enthalten ist.

Weitere besonders bevorzugte erfindungsgemäße lamellare Öl-in-Wasser-Emulsionen sind dadurch gekennzeichnet, dass mindestens ein Alkyl- und/oder Alkoxy-substituiertes Dibenzoylmethanderivat, mindestens ein wasserlöslicher UV-Filter, ausgewählt aus Phenylbenzimidazolsulfonsäure und Phenyldibenzimidazoltetrasulfonsäure und deren physiologisch verträglichen Salzen und/oder Estern, und als weiterer UV-Filter mindestens ein anorganisches UV-Schutz-Pigment enthalten ist, wobei die Emulsion frei ist von Salicylsäureestern.
Erfindungsgemäß bevorzugte anorganische UV-Schutz-Pigmente zeichnen sich durch eine zahlenmittlere Primärpartikelgröße von 5 nm bis 300 nm, bevorzugt 10 - 200 nm, besonders bevorzugt 20 - 150 nm, aus. Besonders bevorzugte anorganische UV-Schutz-Pigmente sind ausgewählt aus Metalloxiden, insbesondere Oxiden des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxiden der entsprechenden Metalle sowie Abmischungen aus solchen Oxiden, sowie Bariumsulfat (BaSO₄). Erfindungsgemäß bevorzugte Titandioxid-Pigmente können sowohl in der Rutil-Modifikation als auch in der Anatas-Modifikation vorliegen.
Weitere erfindungsgemäß bevorzugte UV-Schutz-Pigmente sind oberflächlich behandelt ("gecoatet"), wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können auch Wasser enthalten. Erfindungsgemäß bevorzugte Zusammensetzungen sind dadurch gekennzeichnet, dass die oberflächlich behandelten oder unbehandelten UV-Schutz-Pigmente in Form öliger oder wässriger Vordispersionen eingesetzt werden. Diese Vordispersionen, die auch kommerziell erhältlich sind, können bevorzugt Dispergierhilfmittel und/oder Solubilisationsvermittler enthalten.
In der nachfolgenden Tabelle sind einige kommerziell erhältliche oberflächlich behandelte Titandioxid-UV-Schutz-Pigmente, die gegebenenfalls als Dispersion vorliegen, zusammengestellt.

| **Handelsname** | **Coating** | **zusätzliche Bestandteile der Vordispersion** | **Hersteller** |
|---|---|---|---|
| MT-100TV | Aluminiumhydroxid Stearinsäure | Pulver | Tayca Corporation |
| MT-100Z | Aluminiumhydroxid Stearinsäure | Pulver | Tayca Corporation |
| MT-100F | Stearinsäure Eisenoxid | Pulver | Tayca Corporation |
| MT-500SAS | Alumina, Silica Silikon | Pulver | Tayca Corporation |
| MT-100AQ | Silica Aluminiumhydroxid Alginsäure | - | Tayca Corporation |
| Eusolex T-2000 | Alumina Simethicone | Pulver | Merck KGaA |
| Eusolex TS | Alumina, Stearinsäure - | | Merck KGaA |
| Titandioxid P 25 | None | - | Degussa |
| Titandioxid T 805 (Uvinul TiO₂) | Octyltrimethylsilan | - | Degussa |
| UV-Titan X170 | Alumina Dimethicone | Pulver | Kemira |
| UV-Titan X161 | Alumina, Silica Stearinsäure | Pulver | Kemira |
| Tioveil AQ 10PG | Alumina Silica | Wasser, Propylenglycol | Solaveil Uniqema |
| Mirasun TiW 60 | Alumina Silica | Wasser | Rhone-Poulenc |

Erfindungsgemäß sind besonders bevorzugte Titandioxid-Produkte sind das MT-100 Z und MT-100 TV von Tayca Corporation, Eusolex T-2000 und Eusolex TS von Merck und das Titandioxid T 805 von Degussa.
Auch UV-Schutz-Pigmente auf der Basis von Zinkoxid sind erfindungsgemäß bevorzugt, insbesondere in Form gegebenenfalls kommerziell erhältlicher öliger oder wässriger Vordispersionen. Erfindungsgemäß besonders bevorzugte UV-Schutz-Pigmente auf der Basis von Zinkoxid-Partikeln und Vordispersionen von Zinkoxid-Partikeln zeichnen sich durch eine zahlenmittlere Primärpartikelgröße von < 300 nm aus und sind beispielsweise unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | Symrise |
| MZ 707M 7% | 7% Dimethicone | M. Tayca Corp. |
| Nanox 500 | / | Elementis |
| ZnO Neutral | / | Symrise |

Erfindungsgemäß besonders bevorzugte Zinkoxid-Produkte sind Z-Cote HP1 von der Firma BASF und Zinkoxid NDM von der Firma Symrise.

Erfindungsgemäß besonders bevorzugte lamellare Öl-in-Wasser-Emulsionen sind dadurch gekennzeichnet, dass das mindestens eine anorganische UV-Schutz-Pigment c)iii) hydrophob und/oder hydrophil beschichtet ist.

Weitere erfindungsgemäß besonders bevorzugte lamellare Öl-in-Wasser-Emulsionen sind dadurch gekennzeichnet, dass das mindestens eine anorganische UV-Schutz-Pigment c)iii) in der wässrigen Phase dispergiert ist.

Weitere erfindungsgemäß besonders bevorzugte lamellare Öl-in-Wasser-Emulsionen sind dadurch gekennzeichnet, dass das mindestens eine anorganische UV-Schutz-Pigment c)iii) in der Ölphase dispergiert ist.
Erfindungsgemäß besonders bevorzugte Zusammensetzungen sind dadurch gekennzeichnet, dass sie mindestens ein anorganisches UV-Schutz-Pigment in einer Gesamtmenge von 0,1 Gew.-% bis 10 Gew.-%, bevorzugt 0,5 - 5- 7 Gew.-% und besonders bevorzugt 1 - 1,5 -2,5 Gew.-%, jeweils bezogen auf das Gesamtgewicht der Emulsion, enthalten.

Weiterhin wurde überraschend festgestellt, dass durch die Anwesenheit der lamellaren Flüssigkristallphasen in den erfindungsgemäßen Öl-in-Wasser-Emulsionen auch die Wirkung weiterer kosmetischer Wirkstoffe in synergistischer Weise gesteigert wird.

In einer weiteren bevorzugten Ausführungsform enthalten die erfindungsgemäßen Zusammensetzungen daher zusätzlich mindestens einen weiteren kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Metallsalzen dieser Substanzen, natürlichen Betainverbindungen, Vitaminen, Provitaminen und Vitaminvorstufen der Gruppen A, B, C, E, H und K und den Estern der vorgenannten Substanzen, Flavonoiden und Flavonoid-reichen Pflanzenextrakten, Isoflavonoiden und Isoflavonoid-reichen Pflanzenextrakten, Polyphenolen und Polyphenol-reichen Pflanzenextrakten, Ubichinon und Ubichinol sowie deren Derivaten, Silymarin, Ectoin, hautberuhigenden und feuchtigkeitsspendenden Wirkstoffen sowie Mischungen dieser Wirkstoffe.

Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Taurin, Zink- und Natriumpyroglutamat und Natriumlauroylglutamat.
Der C₂-C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.
Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF- ß (*tissue growth factor*)*,* der die Synthese von Collagen in dermalen Fibroblasten induziert, freizusetzen. Derartige Aminosäureoligomere können als Wirkstoffe gegen die Hautalterung verwendet werden.
Erfindungsgemäß bevorzugte, gegebenenfalls N-acylierte und/oder veresterte Dipeptide sind Acetyl-Citrullyl-Arginin (z. B. Exsy-Algine von Exsymol), Tyr-Arg (Dipeptide-1), Val-Trp (Dipeptide-2), Asn-Phe, Asp-Phe, N-Palmitoyl-ß-Ala-His, N-Acetyl-Tyr-Arg-hexyldecylester (z. B. Calmosensine von Sederma), Carnosin (ß-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn und N-Acetyl-Arg-Lys-Arg-NH₂.
Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).
Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt. ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.
Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes optionales Aminosäureoligomer ist das Peptidderivat L-Glutamylaminoethyl-indol (Glistin von Exsymol).

Die Polymere der Aminosäuren und/oder der N-C₂-C₂₄-Acylaminosäuren sind ausgewählt aus pflanzlichen und tierischen Proteinhydrolysaten und/oder Proteinen. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, z. B. die Handelsprodukte Phytokine von Coletica oder Ridulisse C von Silab. Proteinhydrolysate können naturgemäß auch monomere Aminosäuren und Oligopeptide enthalten; ihre Zusammensetzung ist normalerweise nicht definiert.
Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).
Erfindungsgemäß einsetzbar sind auch kationisierte Proteinhydrolysate. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCl-Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.

In einer weiteren bevorzugten Ausführungsform sind die Polymeren der Aminosäuren ausgewählt aus DNA-Reparaturenzymen.
Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.
Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen. Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosomes™, liposomenverkapselte T4N5 z. B. unter der Bezeichnung Ultrasomes™ von der Firma AGI Dermatics, USA, erhältlich.
In den erfindungsgemäßen Zusammensetzungen sind die Photosome™ oder Ultrasome™ in Mengen von 0,1 - 10 Gew.-%, bevorzugt 0,5 - 5,0 Gew.-% und besonders bevorzugt 1,0 - 4,0 Gew.-%, bezogen auf das gesamte Mittel, enthalten.
In den erfindungsgemäßen Zusammensetzungen sind die Monomere, Oligomere oder Polymere von Aminosäuren, N-C₂-C₂₄-Acylaminosäuren und/oder den Estern und/oder den physiologisch verträglichen Metallsalzen dieser Substanzen in Mengen von 0,00001 - 5 Gew.-%, bevorzugt 0,001 - 3 Gew.-% und besonders bevorzugt 0,01 - 1 Gew.-%, jeweils bezogen auf die gesamte Zusammensetzung, enthalten.

In einer weiteren bevorzugten Ausführungsform liegen die Monomeren, Oligomeren und Polymeren von Aminosäuren, N-C₂-C₂₄-Acylaminosäuren, den Estern und/oder den physiologisch verträglichen Metallsalzen dieser Substanzen in geträgerter Form vor, insbesondere aufgetragen auf feinteiligen, pulverförmigen Substraten wie Kieselgel, insbesondere Aerosil-Typen, Talkum, Microsponges, modifizierten Stärken und Stärkederivaten, kristalliner Cellulose, Cellulosepulvern, Lactoglobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

In einer weiteren bevorzugten Ausführungsform enthalten die erfindungsgemäßen Zusammensetzungen zusätzlich mindestens ein DNA-Oligonucleotid oder ein RNA-Oligonucleotid.
Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).
Ein erfindungsgemäß besonders bevorzugtes Oligonucleotid ist das Thymidin-Dinucleotid. In den erfindungsgemäßen Zusammensetzungen sind die DNA-Oligonucleotide oder RNA-Oligonucleotide in Mengen von 0,00001 - 5 Gew.-%, bevorzugt 0,0001 - 1,0 Gew.-% und besonders bevorzugt 0,0005 - 0,5 Gew.-%, bezogen auf das gesamte Mittel, enthalten.
In einer weiteren bevorzugten Ausführungsform enthalten die erfindungsgemäßen Zusammensetzungen zusätzlich mindestens eine natürliche Betainverbindung. Erfindungsgemäß eingesetzte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl.
Die Betainverbindungen sind in den erfindungsgemäßen Zusammensetzungen in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen zusätzlich mindestens ein Vitamin, Provitamin oder eine als Vitaminvorstufe bezeichnete Verbindung aus den Vitamingruppen A, B, C, E, H und K oder den Estern der vorgenannten Substanzen.
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen Zubereitungen enthalten die Vitamin A-Komponente bevorzugt in Mengen von 0,05 - 1 Gew.-%, bezogen auf die gesamte Zusammensetzung.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf das gesamte Mittel, eingesetzt.
- Vitamin B₂, Trivialrame Riboflavin, chemische Bezeichung 7,8-Dimethyl-10-(1-D-ribityl)-benzo[g]pteridin-2,4(3H,10H)-dion. Bevorzugt werden Riboflavin oder seine Derivate in Mengen von 0,05 bis 1 Gew.-%, bezogen auf das gesamte Mittel, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.

- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden.

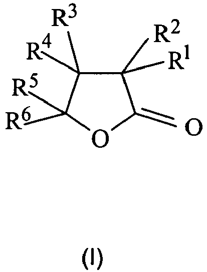

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄-Kohlenwasserstoffrest, einen gesättigten oder ein- oder zweifach ungesättigten, verzweigten oder linearen Mono-, Di- oder Trihydroxy-C₂-C₄-Kohlenwasserstoffrest oder einen gesättigten oder ein- oder zweifach ungesättigten, verzweigten oder linearen Mono-, Di- oder Triamino-C₂-C₄-Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-y-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.

Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3aS,4S, 6a*R*)-2-Oxohexahydrothienol[3,4-d]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

Vitamin C (Ascorbinsäure) wird bevorzugt in Mengen von 0,1 bis 3 Gew.-%, bezogen auf die gesamte Zusammensetzung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.
Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.
Unter Vitamin F werden üblicherweise essentielle Fettsäuren, insbesondere Linolsäure, Linolensäure und Arachidonsäure, verstanden. Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.
Vitamin A-palmitat (Retinylpalmitat), Panthenol, Pantolacton, Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen zusätzlich mindestens ein Flavonoid oder einen Flavonoid-reichen Pflanzenextrakt. Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid). Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.
Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten Zusammensetzung, eingesetzt.

In einer weiteren bevorzugten Ausführungsform enthalten die erfindungsgemäßen Zusammensetzungen zusätzlich mindestens ein Isoflavonoid oder einen Isoflavonoid-reichen Pflanzenextrakt. Zu den Isoflavonoiden werden an dieser Stelle die Isoflavone und die Isoflavon-Glycoside gezählt. Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß werden die Isoflavonoide in Mengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten Zusammensetzung, eingesetzt.

In einer weiteren bevorzugten Ausführungsform enthalten die erfindungsgemäßen Zusammensetzungen zusätzlich mindestens ein Polyphenol oder einen Polyphenol-reichen Pflanzenextrakt. Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay, ein Extrakt aus den Kernen der Chardonnay-Traube, erhältlich von der Firma Croda.
Erfindungsgemäß werden die Polyphenole in Mengen von 0,001 bis 10 Gew.-%, bevorzugt 0,005 bis 5 Gew.-% und besonders bevorzugt 0,01 bis 3 Gew.-%, jeweils bezogen auf die gesamte Zusammensetzung, eingesetzt.

In einer weiteren bevorzugten Ausführungsform enthalten die erfindungsgemäßen Zusammensetzungen zusätzlich mindestens ein Ubichinon oder Ubichinol oder deren Derivate. Ubichinole sind die reduzierte Form der Ubichinone. Die erfindungsgemäß bevorzugten Ubichinone weisen die Formel (II) auf: 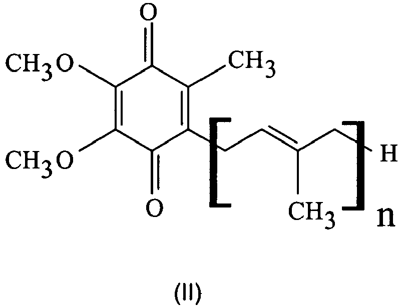 mit n = 6, 7, 8, 9 oder 10.
Besonders bevorzugt ist das Ubichinon der Formel (II) mit n = 10, auch bekannt als Coenzym Q10.
Erfindungsgemäß werden die Ubichinone, Ubichinole oder deren Derivate in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

In einer weiteren bevorzugten Ausführungsform enthalten die erfindungsgemäßen Zusammensetzungen zusätzlich Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Silymarin wird besonders bevorzugt in liposomenverkapselter Form eingesetzt, erhältlich z. B. unter der Handelsbezeichnung Silymarin Phytosome (INCI: Silybum Marianum Extract and Phospholipids) von der Firma Indena SpA.
Erfindungsgemäß wird Silymarin in Mengen von 0,0001 bis 1 Gew.-%, bevorzugt 0,001 bis 0,5 Gew.-% und besonders bevorzugt 0,005 bis 0,1 Gew.-%, jeweils bezogen auf die Aktivsubstanz in der gesamten Zusammensetzung, eingesetzt.

In einer weiteren bevorzugten Ausführungsform enthalten die erfindungsgemäßen Zusammensetzungen zusätzlich Ectoin. Ectoin ist der Trivialname für 2-Methyl-1,4,5,6-tetrahydropyrimidin-4-carboxylat. Erfindungsgemäß ist Ectoin in Mengen von 0,0001 bis 1 Gew.-%, bevorzugt 0,001 bis 0,5 Gew.-% und besonders bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen zusätzlich mindestens einen hautberuhigenden oder feuchtigkeitsspendenden Wirkstoff.
Erfindungsgemäß bevorzugte hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter der Handelsbezeichnung Calmsphere von Soliance erhältlich ist, Mischungen aus Getreidewachsen, Extrakten aus Schibutter und Argania Spinosa-Öl mit der INCI-Bezeichnung "Spent grain wax and butyrospermum parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Extrakten aus Olivenblättern (INCl: Olea Europaea (Olive) Leaf Extract), wie sie insbesondere unter der Handelsbezeichnung Oleanoline DPG von der Firma Vincience erhältlich sind, weiterhin Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter der Handelsbezeichnung Rooibos Herbasec MPE von der Firma Cosmetochem erhältlich sind, den physiologisch verträglichen Salzen von Sterolsulfaten, wie sie z. B. unter der Handelsbezeichnung Phytocohesine (INCI: Sodium Beta-Sitosterylsulfate) von der Firma Vincience erhältlich sind, sowie beliebigen Mischungen dieser Wirkstoffe.
Die hautberuhigenden Wirkstoffe sind in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.
Erfindungsgemäß bevorzugte feuchtigkeitsspendende Wirkstoffe sind ausgewählt aus Desoxyzuckern, besonders bevorzugt Rhamnose und Fucose, Polysacchariden, die mindestens einen Desoxyzucker-Baustein enthalten, besonders bevorzugt das Handelsprodukt Fucogel^{®}, Harnstoff, (2-Hydroxyethyl)harnstoff, Glycosaminoglycanen, besonders bevorzugt Hyaluronsäure, Dextran, Dextransulfat, Chondroitin-4-sulfat und Chondroitin-6-sulfat.
Die feuchtigkeitsspendenden Wirkstoffe sind in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

Die folgenden Beispiele sollen die Erfindung erläutern, ohne sie hierauf zu beschränken.

**1. Beispielserie:**

| | 1 | 2 | 3 | 4 | 5 | 6 | 7 |
|---|---|---|---|---|---|---|---|
| Montanov 68 | 5,00 | 5,00 | 5,00 | 5,00 | 5,00 | 5,00 | 5,00 |
| Myritol 318 | 5,00 | 5,00 | 5,00 | 5,00 | 5,00 | 5,00 | 5,00 |
| Cetiol SB 45 | 0,50 | 0,50 | 0,50 | 0,50 | 0,50 | 0,50 | 0,50 |
| Novata AB | 2,00 | 2,00 | 2,00 | 2,00 | 2,00 | 2,00 | 2,00 |
| Stenol 1618 | 1,00 | 1,00 | 1,00 | 1,00 | 1,00 | 1,00 | 1,00 |
| Cutina MD | 2,00 | 2,00 | 2,00 | 2,00 | 2,00 | 2,00 | 2,00 |
| Baysilon M 350 | 0,50 | 0,50 | 0,50 | 0,50 | 0,50 | 0,50 | 0,50 |
| Tocopherylacetat | 0,50 | 0,50 | 0,50 | 0,50 | 0,50 | 0,50 | 0,50 |
| Controx KS | 0,25 | 0,25 | 0,25 | 0,25 | 0,25 | 0,25 | 0,25 |
| Parsol SLX | 4,00 | 3,00 | 4,00 | 4,00 | 4,00 | 4,00 | 5,00 |
| Parsol KS | 2,00 | - | - | 3,00 | - | - | - |
| Uvinul T 150 | - | - | 2,00 | - | - | 2,00 | 2,00 |
| Solaveil CT 100 | - | 2,00 | - | - | 2,00 | - | - |
| Parsol 1789 | 1,80 | 2,50 | 1,80 | 1,00 | 1,80 | 2,50 | 1,80 |
| Propylparaben | 0,20 | 0,20 | 0,20 | 0,20 | 0,20 | 0,20 | 0,20 |
| Tego Carbomer 140 | 0,50 | 0,50 | 0,50 | 0,50 | 0,50 | 0,50 | 0,50 |
| Hexandiol | 6,00 | 6,00 | 3,00 | 6,00 | - | - | - |
| Talkum Pharma G | 0,50 | 0,50 | 0,50 | 0,50 | 0,50 | 0,50 | 0,50 |
| Methylparaben | 0,20 | 0,20 | 0,20 | 0,20 | 0,20 | 0,20 | 0,20 |
| Dry Flo Plus | 1,00 | 1,00 | 1,00 | 1,00 | 1,00 | 1,00 | 1,00 |
| Glycerin 86 % | 4,50 | 4,50 | 3,00 | 4,50 | 3,00 | 3,00 | 3,00 |
| Simulgel EPG | 2,00 | 2,00 | 2,00 | 2,00 | 2,00 | 2,00 | 2,00 |
| Trilon A | 0,10 | 0,10 | 0,10 | 0,10 | 0,10 | 0,10 | 0,10 |
| Phenoxyethanol | 0,40 | 0,40 | 0,40 | 0,40 | 0,40 | 0,40 | 0,40 |
| Liftiline | 3,00 | 3,00 | 3,00 | - | - | - | - |
| Gatuline R/C | 2,00 | 2,00 | 2,00 | - | - | - | - |
| Phytokine | 2,00 | 2,00 | 2,00 | - | - | - | - |
| Natipide II | 1,00 | 1,00 | 1,00 | - | - | - | - |
| Matrixyl 3000 | - | - | - | 3,00 | 3,00 | 3,00 | - |
| Ederline L | - | - | - | 2,00 | 2,00 | 2,00 | - |
| Silymarin Phytosome | - | - | - | 0,20 | 0,20 | 0,20 | - |
| Aqua | ad 100 | ad 100 | ad 100 | ad 100 | ad 100 | ad 100 | ad 100 |

**2. Beispielserie:**

| | 1 | 2 | 3 | 4 | 5 | 6 | 7 |
|---|---|---|---|---|---|---|---|
| Montanov 202 | 5,00 | 5,00 | 5,00 | 5,00 | 5,00 | 5,00 | 5,00 |
| Stenol 1618 | 1,00 | 1,00 | 1,00 | 1,00 | 1,00 | 1,00 | 1,00 |
| Cutina MD | 2,00 | 2,00 | 2,00 | 2,00 | 2,00 | 2,00 | 2,00 |
| Cetiol B | 9,00 | 9,00 | 9,00 | 9,00 | 9,00 | 9,00 | 9,00 |
| Controx KS | 0,05 | 0,05 | 0,05 | 0,05 | 0,05 | 0,05 | 0,05 |
| Propylparaben | 0,20 | 0,20 | 0,20 | 0,20 | 0,20 | 0,20 | 0,20 |
| Methylparaben | 0,20 | 0,20 | 0,20 | 0,20 | 0,20 | 0,20 | 0,20 |
| DC 9040 Fluid | 1,00 | 1,00 | 1,00 | 1,00 | 1,00 | 1,00 | 1,00 |
| Tego Carbomer 140 | 15,00 | 15,00 | 15,00 | 15,00 | 15,00 | 15,00 | 15,00 |
| Glycerin 86 % | 3,00 | 3,00 | 3,00 | 3,00 | 3,00 | 3,00 | 3,00 |
| 1,6-Hexandiol | 6,00 | 6,00 | 3,00 | 6,00 | 6,00 | 6,00 | 6,00 |
| 1,2-Propylenglycol | 2,00 | 2,00 | 2,00 | 2,00 | 2,00 | 2,00 | 2,00 |
| DSH-CN | 5,00 | 5,00 | 5,00 | 5,00 | 5,00 | 5,00 | 5,00 |
| Dry Flo PLus | 2,00 | 2,00 | 2,00 | 2,00 | 2,00 | 2,00 | 2,00 |
| Simulgel EPG | 1,00 | 1,00 | 1,00 | 1,00 | 1,00 | 1,00 | 1,00 |
| Parsol SLX | 4,00 | 4,00 | 4,00 | 4,00 | 4,00 | 4,00 | 5,00 |
| Parsol HS | 2,00 | - | - | 2,00 | - | - | - |
| Uvinul T 150 | - | - | 2,00 | - | - | 2,50 | 2,00 |
| Solaveil CT 100 | - | 2,00 | - | - | 2,00 | | |
| Parsol 1789 | 1,80 | 1,80 | 1,80 | 1,80 | 1,80 | 1,80 | 2,50 |
| Liftiline | 3,00 | 3,00 | 3,00 | - | - | - | - |
| Gatuline R/C | 2,00 | 2,00 | 2,00 | - | - | - | - |
| Phytokine | 2,00 | 2,00 | 2,00 | - | - | - | - |
| Natipide II | 1,00 | 1,00 | 1,00 | - | - | - | - |
| Matrixyl 3000 | - | - | - | - | 3,00 | 3,00 | - |
| Ederline L | - | - | - | - | 2,00 | 2,00 | - |
| Silymarin Phytosome | - | - | - | - | 0,20 | 0,20 | - |
| Caomint | - | - | - | 1,00 | - | - | - |
| Calmosensine | - | - | - | - | - | 2,00 | - |
| Aqua | ad 100 | ad 100 | ad 100 | ad 100 | ad 100 | ad 100 | ad 100 |

**3. Beispielserie:**

| | 1 | 2 | 3 | 4 | 5 | 6 | 7 |
|---|---|---|---|---|---|---|---|
| Montanov 68 | 5,00 | 5,00 | 5,00 | 5,00 | 5,00 | 5,00 | 5,00 |
| Myritol 318 | 5,00 | 5,00 | 5,00 | 5,00 | 5,00 | 5,00 | 5,00 |
| Cetiol SB 45 | 0,50 | 0,50 | 0,50 | 0,50 | 0,50 | 0,50 | 0,50 |
| Novata AB | 2,00 | 2,00 | 2,00 | 2,00 | 2,00 | 2,00 | 2,00 |
| Stenol 1618 | 1,00 | 1,00 | 1,00 | 1,00 | 1,00 | 1,00 | 1,00 |
| Cutina MD | 2,00 | 2,00 | 2,00 | 2,00 | 2,00 | 2,00 | 2,00 |
| Baysilon M 350 | 0,50 | 0,50 | 0,50 | 0,50 | 0,50 | 0,50 | 0,50 |
| Tocopherylacetat | 0,50 | 0,50 | 0,50 | 0,50 | 0,50 | 0,50 | 0,50 |
| Controx KS | 0,25 | 0,25 | 0,25 | 0,25 | 0,25 | 0,25 | 0,25 |
| Parsol SLX | 4,00 | 4,00 | 4,00 | 4,00 | 4,00 | 4,00 | 4,00 |
| Parsol HS | 2,00 | - | - | 2,00 | - | - | - |
| Uvinul T 150 | - | - | 2,00 | - | - | 2,50 | 2,00 |
| Solaveil CT 100 | - | 2,00 | - | - | 1,50 | - | - |
| Parsol 1789 | 1,80 | 1,80 | 1,80 | 1,80 | 2,00 | 1,80 | 1,80 |
| Propylparaben | 0,20 | 0,20 | 0,20 | 0,20 | 0,20 | 0,20 | 0,20 |
| Tego Carbomer 140 | 0,50 | 0,50 | 0,50 | 0,50 | 0,50 | 0,50 | 0,50 |
| 1,6-Hexandiol | 6,00 | 6,00 | 3,00 | 6,00 | 6,00 | 6,00 | 6,00 |
| Talkum Pharma G | 0,50 | 0,50 | 0,50 | 0,50 | 0,50 | 0,50 | 0,50 |
| Methylparaben | 0,20 | 0,20 | 0,20 | 0,20 | 0,20 | 0,20 | 0,20 |
| Dry Flo Plus | 1,00 | 1,00 | 1,00 | 1,00 | 1,00 | 1,00 | 1,00 |
| Glycerin 86 % | 4,50 | 4,50 | 3,00 | 4,50 | 3,00 | 3,00 | 3,00 |
| Simulgel EPG | 2,00 | 2,00 | 2,00 | 2,00 | 2,00 | 2,00 | 2,00 |
| Trilon A | 0,10 | 0,10 | 0,10 | 0,10 | 0,10 | 0,10 | 0,10 |
| Phenoxyethanol | 0,40 | 0,40 | 0,40 | 0,40 | 0,40 | 0,40 | 0,40 |
| Taurin | - | 1,00 | - | - | - | - | - |
| Coenzyme Q10 | 0,20 | - | - | - | - | - | - |
| Kreatin | 1,00 | - | - | - | - | 1,00 | - |
| Ultrasomes | 0,10 | - | 0,10 | - | - | - | - |
| Photosomes | - | 0,10 | - | - | - | - | - |
| Kombuchka | - | 3,00 | - | - | - | - | - |
| Herbalia Grape | - | - | 0,50 | 0,50 | 0,50 | - | 0,50 |
| Silymarin-Phytosomes | - | - | - | 1,00 | | - | - |
| Vitamin C Phosphat | 0,50 | - | 0,50 | 0,50 | 0,50 | - | 0,50 |
| Chrodarom Chardonnay | - | 0,50 | - | - | | 0,50 | - |
| Greenselect Phytosome | - | - | - | - | 1,00 | - | - |
| SynColl | - | - | - | 3,00 | - | - | - |
| Sepivinol R | - | - | 0,01 | - | - | - | 0,01 |
| L-Carnitin | 1,00 | - | - | 1,00 | 1,00 | 1,00 | 1,00 |
| Algenextrakt SPHM 3002 | - | 1,00 | - | - | 1,00 | - | - |
| Aqua | ad 100 | ad 100 | ad 100 | ad 100 | ad 100 | ad 100 | ad 100 |

**1. Lamellare Öl-in-Wasser-Emulsionen als Tagescremes**

| | 1.1 | 1.2 |
|---|---|---|
| Montanov 202 | 5,00 | 5,00 |
| Cutina MD-V | 2,00 | 2,00 |
| Stenol 16/18 | 1,00 | 1,00 |
| Cetiol B | 9,00 | 9,00 |
| Propylparaben | 0,20 | 0,20 |
| Methylparaben | 0,20 | 0,20 |
| Controx KS | 0,05 | 0,05 |
| Dow Corninq 9040 | 1,00 | 1,00 |
| Glycerin 86% | 3,00 | 3,00 |
| Propylenglycol | 2,00 | 2,00 |
| Hexandiol | 5,00 | 5,00 |
| Tego Carbomer 140 (2 %ige Quellung) | 15,00 | 15,00 |
| Dry Flo Plus | 2,00 | 2,00 |
| Simulgel NS | 1,00 | 1,00 |
| Taurin | 1,00 | 0,50 |
| Biopeptide CL | 1,00 | 0,50 |
| Betafin BP 20 | - | 1,00 |
| Wasser | ad 100 | ad 100 |

**2. Lamellare Öl-in-Wasser-Emulsionen als Tagescremes**

| | 2.1 | 2.2 |
|---|---|---|
| Distelöl | 3,00 | 3,00 |
| Myritol 318 | 5,00 | 5,00 |
| Novata AB | 2,00 | 2,00 |
| Lanette 22 | 1,00 | 1,00 |
| Cutina MD | 2,00 | 2,00 |
| Stenol 16/18 | 1,00 | 1,00 |
| Isopropylstearat | 4,00 | 4,00 |
| Cetiol SB 45 | 2,00 | 2,00 |
| Baysilone-Öl M 350 | 1,00 | 1,00 |
| Controx KS | 0,05 | 0,05 |
| Propylparaben | 0,20 | 0,20 |
| Dow Corning 1501 Fluid | 1,00 | 1,00 |
| Dry Flo Plus | 1,00 | 1,00 |
| TiO₂ | 0,50 | 0,50 |
| Hexandiol | 6,00 | 3,00 |
| Propylenglycol | 5,00 | 5,00 |
| Glycerin 86 % | 5,00 | 3,00 |
| Methylparaben | 0,20 | 0,20 |
| Tego Carbomer 140 | 0,40 | 0,40 |
| DSH-CN | 5,00 | 5,00 |
| Taurin | 1,00 | 0,50 |
| Sepivinol R | 1,50 | - |
| Biopeptide EL | 1,00 | 0,50 |
| Parfum | 0,10 | 0,10 |
| Wasser | ad 100 | ad 100 |

**3. Lamellare Öl-in-Wasser-Emulsionen mit DNA-Reparaturenzym und Silymarin**

| | 3.1 | 3.2 |
|---|---|---|
| Lipoid S75-3 | 0,50 | 0,50 |
| Isopropylstearat | 4,00 | 4,00 |
| Cetiol B | 2,00 | 2,00 |
| Tocopherylacetat | 0,50 | 0,50 |
| Cutina MD | 1,00 | 1,00 |
| Lanette 22 | 2,00 | 2,00 |
| Baysilon M 350 | 0,50 | 0,50 |
| Propylparaben | 0,20 | 0,20 |
| Dow Corning 9040 | 1,00 | 1,00 |
| Glycerin 86 % | 4,50 | 3,00 |
| Hexandiol | 6,00 | 3,00 |
| Methylparaben | 0,20 | 0,20 |
| Tego Carbomer 140 | 0,30 | 0,30 |
| DSH-CN | 5,00 | 5,00 |
| Alqenextrakt | 1,00 | - |
| Photosomes | 0,20 | 0,20 |
| Lipochroman-6 | 0,01 | 0,01 |
| Propylenglycol | 5,00 | 5,00 |
| Matrixyl | 3,00 | - |
| Matrixyl 3000 | - | 3,00 |
| Simulgel NS | 1,50 | 1,50 |
| TiO₂ | 0,50 | 0,50 |
| Taurin | 1,00 | 0,50 |
| Calmosensine | 1,00 | 0,50 |
| Silymarin Phytosome | - | 2,00 |
| Parfum | 0,35 | 0,35 |
| Wasser | ad 100 | ad 100 |

**4. Lamellare Öl-in-Wasser-Emulsionen mit Sonnenschutz**

| | 4.1 | 4.2 | 4.3 | 4.4 | 4.5 |
|---|---|---|---|---|---|
| Montanov 68 | 5,00 | 5,00 | 5,00 | 5,00 | 5,00 |
| Myritol 318 | 5,00 | 5,00 | 5,00 | 5,00 | 5,00 |
| Cetiol SB 45 | 0,50 | 0,50 | 0,50 | 0,50 | 0,50 |
| Novata AB | 2,00 | 2,00 | 2,00 | 2,00 | 2,00 |
| Stenol 16/18 | 1,00 | 1,00 | 1,00 | 1,00 | 1,00 |
| Lanette 22 | 1,00 | 1,00 | 1,00 | 1,00 | 1,00 |
| Baysilon M 350 | 0,50 | 0,50 | 0,50 | 0,50 | 0,50 |
| Tocopherylacetat | 0,50 | 0,50 | 0,50 | 0,50 | 0,50 |
| Controx KS | 0,25 | 0,25 | 0,25 | 0,25 | 0,25 |
| Parsol SLX | - | 4,00 | 4,00 | - | - |
| Parsol HS | - | 2,00 | 2,00 | 2,00 | - |
| Neo Heliopan AP | - | - | - | 1,00 | - |
| Parsol 340 | - | - | - | 5,00 | - |
| Parsol 1789 | 1,00 | 1,80 | 1,80 | - | - |
| Propylparaben | 0,20 | 0,20 | 0,20 | 0,20 | 0,20 |
| Tego Carbomer 140 | 0,50 | 0,50 | 0,50 | 0,50 | 0,50 |
| Hexandiol | 6,00 | 6,00 | 3,00 | 6,00 | - |
| Talkum Pharma G | 0,50 | 0,50 | 0,50 | 0,50 | 0,50 |
| Methylparaben | 0,20 | 0,20 | 0,20 | 0,20 | 0,20 |
| Glycerin 86 % | 4,50 | 4,50 | 3,00 | 4,50 | 3,00 |
| Dry Flo Plus | 1,00 | 1,00 | 1,00 | 1,00 | 1,00 |
| Natipide 2 PG | 1,00 | 1,00 | 1,00 | 1,00 | 1,00 |
| Gatuline R/C | 2,00 | 2,00 | 2,00 | 2,00 | 2,00 |
| DSH-CN | 2,00 | 2,00 | 2,00 | 2,00 | 2,00 |
| Trilon A | 0,10 | 0,10 | 0,10 | 0,10 | 0,10 |
| Phenoxyethanol | 0,40 | 0,40 | 0,40 | 0,40 | 0,40 |
| Taurin | 1,00 | 0,40 | 1,00 | 1,00 | 0,50 |
| Omega-CH-Aktivator | 0,50 | - | - | 0,80 | 1,00 |
| Soy Protein Isolate | - | - | 1,00 | - | 1,50 |
| Panthenol | 0,50 | - | - | 0,50 | - |
| Parfum | 0,40 | 0,40 | 0,40 | 0,40 | 0,40 |
| Wasser | ad 100 | ad 100 | ad 100 | ad 100 | ad 100 |

**5. Lamellare Öl-in-Wasser-Emulsionen**

| | 5.1 | 5.2 | 5.3 |
|---|---|---|---|
| Montanov L | 3,00 | 3,00 | 3,00 |
| Cetiol B | 6,00 | 6,00 | 6,00 |
| Myritol 318 | 3,00 | 3,00 | 3,00 |
| Stenol 1618 | 1,00 | 1,00 | 1,00 |
| Cutina MD | 0,50 | 0,50 | 0,50 |
| Baysilon M 350 | 0,50 | 0,50 | 0,50 |
| Propylparaben | 0,20 | 0,20 | 0,20 |
| Dow Corninq 245 | 1,50 | 1,50 | 1,50 |
| Glycerin 86 % | 5,00 | 5,00 | 5,00 |
| Karion F | 3,00 | 3,00 | 3,00 |
| Methylparaben | 0,20 | 0,20 | 0,20 |
| Dry Flo Plus | 0,50 | 0,50 | 0,50 |
| Tego Carbomer 140 | 0,10 | 0,10 | 0,10 |
| DSH-CN | 2,00 | 2,00 | 2,00 |
| Seanerqilium | 0,50 | 0,50 | 0,50 |
| Simulgel NS | 1,00 | 1,00 | 1,00 |
| Taurin | 1,00 | 0,50 | 1,00 |
| Matrixyl | - | 1,00 | - |
| Retinylpalmitat | 0,20 | - | 0,20 |
| Oleanoline DPG | - | 2,00 | 1,00 |
| Parfum | 0,20 | 0,20 | 0,20 |
| Wasser | ad 100 | ad 100 | ad 100 |

**6. Lamellare Öl-in-Wasser-Emulsionen**

| | 6.1 | 6.2 |
|---|---|---|
| Paraffinum Liquidum | 20,00 | 20,00 |
| Hostaphat KW 340 D | 2,50 | 2,50 |
| Cetearyl Alcohol | 1,40 | 1,40 |
| Lanette 22 | 1,00 | 1,00 |
| Eumulqin B 1 | 1,70 | 1,70 |
| Vitamin E Acetat | 0,25 | 0,25 |
| Propylparaben | 0,20 | 0,20 |
| Glycerin 86% | 2,00 | 2,00 |
| Hexandiol-1,6 | 5,00 | 5,00 |
| Sorbit 70% LS DAB | 1,00 | 1,00 |
| Taurin | 0,50 | 1,00 |
| Methylparaben | 0,20 | 0,20 |
| Tego Carbomer 140 | 0,30 | 0,30 |
| Keltrol SF | 0,10 | 0,10 |
| Benecel MP 333C | 0,24 | 0,24 |
| D-Panthenol 75 % | 0,66 | 0,66 |
| Phenoxyethanol | 0,40 | 0,40 |
| Calcium D-Pantothenat | 0,05 | 0,05 |
| Matrixyl 3000 | 2,00 | 1,00 |
| Natriumascorbylphosphat | - | 0,50 |
| Wasser | ad 100 | ad 100 |

**Liste der verwendeten Rohstoffe**

| Handelsname | INCl-Bezeichnung | Lieferant/Hersteller |
|---|---|---|
| | | |
| Baysilone-Öl M 350 | Dimethicone | GE Bayer Silicones |
| Benecel MP 333 C | HYDROXYPROPYL METHYLCELLULOSE | Hercules |
| Betafin BP 20 | Betaine | Danisco |
| Biopeptide CL | N-Palmitoyl-Gly-His-Lys | Sederma |
| Biopeptide EL | N-Palmitoyl-Val-Gly-Val-Ala-Pro-Gly | Sederma |
| Calmosensine | Acetyl Dipeptide-1 Cetylester | Sederma |
| Caomint | Propylene Glycol, Aqua, Mentha piperita, Theobroma cacao | Solabia |
| Cetiol^{®} B | Dibutyl Adipate | Coqnis |
| Cetiol^{®} SB 45 | Butyrospermum Parkii (Shea Butter) | Cognis |
| Controx KS | Tocopherol, Hydrogenated Palm Glycerides Citrate | Cognis |
| Cutina MD-V | Glyceryl Stearate (vegetal) | Coqnis |
| Dow Corning 1501 Fluid | Cyclomethicone, Dimethiconol (83 % Decamethylcyclopentasiloxan, 15 - 17 % Dimethiconol) | Dow Corning |
| Dow Corning 9040 | Cyclomethicone/ Dimethicone Crosspolymer | Dow Corning |
| Dow Corning^{®}245 | Cyclomethicone | Dow Corning |
| Dry Flo Plus | Aluminium Starch Octenylsuccinate | National Starch |
| DSH CN | Water, Dimethylsilanol Hyaluronate | Exsymol |
| Ederline L | Hexyldecanol, Pyrus Malus (Apple) Fruit Extract | Seporga |
| Eumulgin B 1 | Ceteareth-12 | Cognis |
| Gatuline R/C | Water, Fagus Sylvatica Extract | Gattefosse |
| Hostaphat^{®} KW 340 | TRICETEARETH-4 PHOSPHATE | Clariant |
| Karion F | Sorbitol (70%), Water | Merck |
| Keltrol SF | Xanthan Gum | CP Kelco |
| Lanette^{®} 22 | Behenyl Alcohol | Cognis |
| Liftiline | Aqua, Hydrolyzed Wheat Protein (7 - 10 Gew.-% Aktivsubstanz) | Silab |
| Lipochroman 6 | Dimethylmethoxy Chromanol | Lipotec |
| Lipoid S 75-3 | Hydrogenated Lecithin | Lipoid GmbH |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 202 | Arachidyl Alcohol, Behenyl Alcohol, Arachidyl Glucoside | Seppic |
| Montanov 68 | Cetearyl Alcohol, Cetearyl Glucoside | Seppic |
| Montanov L | C14-22 Alcohols/C12-20 Alkyl Glucoside | Seppic |
| Mvritol 318 | Caprylic/Capric Triglyceride | Cognis |
| Natipide II | Aqua (Water), Alcohol, Lecithin | Nattermann |
| Neo Heliopan AP | Disodium Phenyl Dibenzimidazole Tetrasulfonate | Symrise |
| Novata AB | Cocoglycerides | Cognis |
| Oleanoline DPG | Dipropylene Glycol, Olea Europaea (Olive) Leaf Extract | Vincience |
| Omega-CH-Aktivator | Tripeptide-1 | GfN |
| Parsol 1789 | Butyl Methoxydibenzoylmethane | Roche |
| Parsol 340 | Octocrylene | Roche |
| Parsol HS | Phenylbenzimidazole sulfonic acid | Roche |
| Parsol SLX | Polysilicone-15 | Roche |
| Photosomes | Aqua (Water), Lecithin, Plancton Extract | AGI Dermatics (Barnet) |
| Phytokine | Water, HYDROLYZED SOY PROTEIN (0,4 - 0,8 Gew.-% Aktivsubstanz) | Coletica |
| Seanergilium | LAMINARIA DIGITATA EXTRACT, BUTYLENE GLYCOL, METHYLPARABEN | Coletica |
| Sepivinol R | Polyphenolreicher Extrakt aus Rotwein | Seppic |
| Silymarin Phytosome | Silybum Marianum Extract and Phospholipids | Indena SpA |
| Simulgel EPG | Hydroxyethyl Acrylate / Sodium Acryloyldimethyl Taurate Copolymer / Polyisobutene / CAPRYLYL/CAPRYL GLUCOSIDE | Seppic |
| Simulgel NS | Hydroxyethyl Acrylate / Sodium Acryloyldimethyl Taurate Copolymer / Squalane / Polysorbate 60 | Seppic |
| Solaveil CT 100 | C12-15 Alkyl Benzoate and Titanium Dioxide and Aluminium Stearate and Polyhydroxystearic Acid and Alumina | Uniqema |
| Soy Protein Isolate | Isoflavonoidreicher Extrakt aus Soja | Protein Technology International |
| Stenol 16/18 | Cetearyl Alcohol | Coqnis |
| Tego Carbomer 140 | Carbomer | Goldschmidt |
| Trilon A | Trisodium NTA | BASF |
| Uvinul T 150 | Ethylhexyl Triazone | BASF |

## Patentansprüche

1. Öl-in-Wasser-Emulsion, enthaltend
a) mindestens ein Alkyl- und/oder Alkoxy-substituiertes Dibenzoylmethanderivat,
b) mindestens einen wasserlöslichen UV-Filter, ausgewählt aus Phenylbenzimidazolsulfonsäure und Phenyldibenzimidazoltetrasulfonsäure und deren physiologisch verträglichen Salzen und/oder Estern,
c) mindestens einen weiteren UV-Filter, ausgewählt aus
i) Polysilicone-15,
ii) s-Triazinderivaten,
iii) mindestens einem anorganischen UV-Schutz-Pigment,
d) mindestens einen primären, linearen, gesättigten Fettalkohol mit einer Kettenlänge von 20 - 40 Kohlenstoffatomen,
**dadurch gekennzeichnet, dass** die Emulsion lamellare flüssig-kristalline Strukturen aufweist und frei ist von Salicylsäureestern.

2. Lamellare Öl-in-Wasser-Emulsion gemäß Anspruch 1, **dadurch gekennzeichnet, dass** Behenylalkohol und/oder Arachidylalkohol enthalten sind.

3. Lamellare Öl-in-Wasser-Emulsion gemäß einem der Ansprüche 1 oder 2, **dadurch gekennzeichnet, dass** sie keine Mikroemulsion und/oder keine PIT-Emulsion ist.

4. Lamellare Öl-in-Wasser-Emulsion gemäß einem der Ansprüche 1 - 3, **dadurch gekennzeichnet, dass** sie frei ist von partiell oder ganz neutralisierten C₆-C₃₀-Fettsäuren.

5. Lamellare Öl-in-Wasser-Emulsion gemäß einem der Ansprüche 1-4, **dadurch gekennzeichnet, dass** sie frei ist von 4-Methylbenzylidencampher.

6. Lamellare Öl-in-Wasser-Emulsion gemäß einem der Ansprüche 1 - 5, **dadurch gekennzeichnet, dass** das Dibenzoylmethanderivat a) ausgewählt ist aus tert.-Butylmethoxydibenzoylmethan.

7. Lamellare Öl-in-Wasser-Emulsion gemäß einem der Ansprüche 1-6, **dadurch gekennzeichnet, dass** das Salz der Phenylbenzimidazolsulfonsäure und/oder Phenyldibenzimidazoltetrasulfonsäure ausgewählt ist aus den Natrium-, Kalium-, Ammonium-, Trialkylammonium- und Ethanolamin-Salzen.

8. Lamellare Öl-in-Wasser-Emulsion gemäß einem der Ansprüche 1-7, **dadurch gekennzeichnet, dass** das Triazinderivat c)ii) ausgewählt ist aus symmetrisch substituierten Triazinderivaten.

9. Lamellare Öl-in-Wasser-Emulsion gemäß Anspruch 8, **dadurch gekennzeichnet, dass** das Triazinderivat c)ii) ausgewählt ist aus Ethylhexyl Triazon.

10. Lamellare Öl-in-Wasser-Emulsion gemäß einem der Ansprüche 1 - 9, **dadurch gekennzeichnet, dass** das mindestens eine anorganische UV-Schutz-Pigment c)iii) eine mittlere Primärpartikelgröße von 5 nm bis 300 nm, bevorzugt 10 - 200 nm, besonders bevorzugt 20 - 150 nm, aufweist.

11. Lamellare Öl-in-Wasser-Emulsion gemäß einem der Ansprüche 1-10, **dadurch gekennzeichnet, dass** das mindestens eine anorganische UV-Schutz-Pigment c)iii) ausgewählt ist aus Titandioxid und Zinkoxid.

12. Lamellare Öl-in-Wasser-Emulsion gemäß einem der Ansprüche 1-11, **dadurch gekennzeichnet, dass** das mindestens eine anorganische UV-Schutz-Pigment c)iii) hydrophob und/oder hydrophil beschichtet ist.

13. Lamellare Öl-in-Wasser-Emulsion gemäß einem der Ansprüche 1 - 12, **dadurch gekennzeichnet, dass** das mindestens eine anorganische UV-Schutz-Pigment c)iii) in der wässrigen Phase dispergiert ist.

14. Lamellare Öl-in-Wasser-Emulsion gemäß einem der Ansprüche 1 - 12, **dadurch gekennzeichnet, dass** das mindestens eine anorganische UV-Schutz-Pigment c)iii) in der Ölphase dispergiert ist.

15. Lamellare Öl-in-Wasser-Emulsion gemäß einem der Ansprüche 1 - 14, **dadurch gekennzeichnet, dass** zusätzlich mindestens ein weiterer kosmetischer Wirkstoff, ausgewählt aus Monomeren, Oligomeren und Polymeren von Aminosäuren, N-C₂-C₂₄-Acylaminosäuren und/oder den Estern und/oder den physiologisch verträglichen Metallsalzen dieser Substanzen, natürlichen Betainverbindungen, Vitaminen, Provitaminen und Vitaminvorstufen der Gruppen A, B, C, E, H und K und den Estern der vorgenannten Substanzen, Flavonoiden und Flavonoid-reichen Pflanzenextrakten, Isoflavonoiden und Isoflavonoid-reichen Pflanzenextrakten, Polyphenolen und Polyphenol-reichen Pflanzenextrakten, Ubichinon und Ubichinol sowie deren Derivaten Silymarin, Ectoin, hautberuhigenden und feuchtigkeitsspendenden Wirkstoffen sowie Mischungen dieser Wirkstoffe, enthalten ist.

## Claims

1. An oil-in-water emulsion, containing
a) at least one alkyl-substituted and/or alkoxy-substituted dibenzoylmethane derivative,
b) at least one water-soluble UV filter selected from phenylbenzimidazole sulfonic acid and phenyl dibenzimidazole tetrasulfonic acid and the physiologically acceptable salts and/or esters thereof,
c) at least one additional UV filter, selected from
i) polysilicones-15
ii) s-triazine derivatives,
iii) at least one inorganic UV-protection pigment,
d) at least one primary, linear, saturated fatty alcohol having a chain length of from 20 to 40 carbon atoms,
**characterized in that** the emulsion comprises lamellar liquid-crystal structures and is free of salicylic acid esters.

2. The lamellar oil-in-water emulsion according to claim 1, **characterized in that** said emulsion contains behenyl alcohol and/or arachidyl alcohol.

3. The lamellar oil-in-water emulsion according to either claim 1 or claim 2, **characterized in that** said emulsion is not a micro-emulsion and/or a PIT emulsion.

4. The lamellar oil-in-water emulsion according to one of claims 1 to 3, **characterized in that** said emulsion is free of partially or fully neutralized C₆-C₃₀ fatty acids.

5. The lamellar oil-in-water emulsion according to one of claims 1 to 4, **characterized in that** said emulsion is free of 4-methylbenzylidene camphor.

6. The lamellar oil-in-water emulsion according to one of claims 1 to 5, **characterized in that** the dibenzoylmethane derivative a) is selected from tert-butyl-methoxydibenzoylmethane.

7. The lamellar oil-in-water emulsion according to one of claims 1 to 6, **characterized in that** the salt of the phenylbenzimidazole sulfonic acid and/or the phenyl dibenzimidazole tetrasulfonic acid is selected from sodium salts, potassium salts, ammonium salts, trialkylammonium salts and ethanolamine salts.

8. The lamellar oil-in-water emulsion according to one of claims 1 to 7, **characterized in that** the triazine derivative c)ii) is selected from symmetrically substituted triazine derivatives.

9. The lamellar oil-in-water emulsion according to claim 8, **characterized in that** the triazine derivative c)ii) is selected from ethylhexyl triazone.

10. The lamellar oil-in-water emulsion according to one of claims 1 to 9, **characterized in that** the at least one inorganic UV-protection pigment c)iii) has an average primary particle size of from 5 nm to 300 nm, preferably from 10 to 200 nm, particularly preferably from 20 to 150 nm.

11. The lamellar oil-in-water emulsion according to one of claims 1 to 10, **characterized in that** the at least one inorganic UV-protection pigment c)iii) is selected from titanium dioxide and zinc oxide.

12. The lamellar oil-in-water emulsion according to one of claims 1 to 11, **characterized in that** the at least one inorganic UV-protection pigment c)iii) has a hydrophobic and/or hydrophilic coating.

13. The lamellar oil-in-water emulsion according to one of claims 1 to 12, **characterized in that** the at least one inorganic UV-protection pigment c)iii) is dispersed in the aqueous phase.

14. The lamellar oil-in-water emulsion according to one of claims 1 to 12, **characterized in that** the at least one inorganic UV-protection pigment c)iii) is dispersed in the oil phase.

15. The lamellar oil-in-water emulsion according to one of claims 1 to 14, **characterized in that** said emulsion additionally contains at least one additional cosmetic active ingredient selected from monomers, oligomers and polymers of amino acids, N-C₂-C₂₄-acyl amino acids and/or the esters and/or the physiologically acceptable metal salts of said substances, natural betaine compounds, vitamins, provitamins and vitamin precursors from groups A, B, C, E, H and K and the esters of said substances, flavanoids and flavanoid-rich plant extracts, isoflavanoids and isoflavanoid-rich plant extracts, polyphenols and polyphenol-rich plant extracts, ubiquinone and ubiquinol and the derivatives thereof silymarin, ectoine, soothing and moisturizing active ingredients and mixtures of said active ingredients.

## Revendications

1. Émulsion huile-dans-eau contenant
a) au moins un dérivé du dibenzoylméthane alkyl- et/ou alcoxy-substitué,
b) au moins un filtre UV hydrosolubles choisis parmi l'acide phénylbenzimidazolsulfonique et l'acide phényldibenzimidazoltétrasulfonique et leurs sels et/ou esters physiologiquement acceptables,
c) au moins un autre filtre UV choisi parmi
i) la polysilicone-15,
ii) la triazine,
iii) au moins un pigment minéral anti-UV,
d) au moins un alcool gras saturé, linéaire, primaire ayant une longueur de chaîne de 20 à 40 atomes de carbone,
**caractérisée en ce que** l'émulsion comporte des structures cristallines liquides lamellaires et est dépourvue d'esters de salicylates.

2. Émulsion huile-dans-eau lamellaire selon la revendication 1, **caractérisée en ce qu'**elle contient de l'alcool béhénylique et/ou arachidylique.

3. Émulsion huile-dans-eau lamellaire selon l'une des revendications 1 ou 2, **caractérisée en ce qu'**elle n'est pas une microémulsion et/ou une émulsion PIT.

4. Émulsion huile-dans-eau lamellaire selon l'une des revendications 1 à 3, **caractérisée en ce qu'**elle est dépourvue d'acides gras en C₆ à C₃₀ partiellement ou totalement neutralisés.

5. Émulsion huile-dans-eau lamellaire selon l'une des revendications 1 à 4, **caractérisée en ce qu'**elle est dépourvue de 4-méthylbenzylidène-camphre.

6. Émulsion huile-dans-eau lamellaire selon l'une des revendications 1 à 5, **caractérisée en ce que** le dérivé de dibenzoylméthane est choisi parmi le tert-butylméthoxydibenzoylméthane.

7. Émulsion huile-dans-eau lamellaire selon l'une des revendications 1 à 6, **caractérisée en ce que** le sel de l'acide phénylbenzimidazolsulfonique et/ou l'acide phényldibenzimidazoltétrasulfonique choisi parmi les sels de sodium, de potassium, d'ammonium, de trialkylammonium, et d'éthanolamine.

8. Émulsion huile-dans-eau lamellaire selon l'une des revendications 1 à 7, **caractérisée en ce que** le dérivé de la triazine c)ii) est choisi parmi les dérivés de la triazine substitués de façon symétrique.

9. Émulsion lamellaire selon huile-dans-eau à la revendication 8, **caractérisée en ce que** le dérivé de triazine c) ii) est choisi parmi l'éthylhexyl triazone.

10. Émulsion huile-dans-eau lamellaire selon l'une des revendications 1 à 9, **caractérisée en ce qu'**au moins un pigment minéral anti-UV c)iii) a une taille moyenne de particules primaires de 5 nm à 300 nm, de préférence de 10 à 200 nm, de façon particulièrement préférée de 20 à 150 nm.

11. Émulsion huile-dans-eau lamellaire selon l'une des revendications 1 à 10, **caractérisée en ce qu'**au moins un pigment minéral anti-UV c)iii) est choisi parmi le dioxyde de titane et l'oxyde de zinc.

12. Émulsion huile-dans-eau selon l'une des revendications 1 à 11, **caractérisée en ce que** l'au moins un pigment minéral anti-UV c)iii) est pourvu d'un revêtement hydrophobe et/ou hydrophile.

13. Émulsion huile-dans-eau lamellaire selon l'une des revendications 1 à 12, **caractérisée en ce qu'**au moins un pigment minéral anti-UV c)iii) est dispersé dans la phase aqueuse.

14. Émulsion huile-dans-eau lamellaire selon l'une des revendications 1 à 12, **caractérisée en ce qu'**au moins un pigment minéral anti-UV c)iii) est dispersé dans la phase huileuse.

15. Émulsion huile-dans-eau lamellaire selon l'une des revendications 1-14, **caractérisée en ce qu'**elle contient en outre au moins une autre substance active cosmétique, choisie parmi les monomères, les oligomères et les polymères d'acides aminés, d'acides N-acylaminés en C₂ à C₂₄ et/ou les esters et/ou les sels métalliques physiologiquement acceptables de ces substances, les composés naturels de la bétaïne, les vitamines, les provitamines et les précurseurs de vitamines des groupes A, B, C, E, H et K et les esters des substances susmentionnées, les flavonoïdes et les extraits de plantes riches en flavonoïdes, les isoflavonoïdes et les extraits de plantes riches en isoflavonoïde, les polyphénols et les extraits de plantes riches en polyphénols, l'ubiquinone et ubiquinol et leurs dérivés, la silymarine, l'ectoïne, les substances apaisantes et hydratantes et des mélanges de ces substances actives.
